Europäisches Patentamt

European Patent Office

Office européen des brevets

(19)

(11) Publication number: **0 031 697**
**A2**

# EUROPEAN PATENT APPLICATION

(21) Application number: **80304640.8**

(22) Date of filing: **19.12.80**

(51) Int. Cl.³: **C 10 B 55/00**, C 07 C 1/00, C 07 C 9/04

(30) Priority: **28.12.79 US 108100**

(43) Date of publication of application: **08.07.81**
**Bulletin 81/27**

(84) Designated Contracting States: **BE CH DE FR GB IT LI LU NL SE**

(71) Applicant: **THE STANDARD OIL COMPANY, Midland Building, Cleveland, Ohio 44115 (US)**

(72) Inventor: **Alford, Harvey Edwin, 177 Orchard Hill Drive, Amherst Ohio 44001 (US)**
Inventor: **Giordano, Paul Joseph, Jr., 75 Bard Drive, Hudson Ohio 44236 (US)**

(74) Representative: **Smith, Sydney et al, Elkington and Fife High Holborn House 52/54 High Holborn, London WC1V 6SH (GB)**

(54) **Improved process for coking petroleum residua and production of methane therefrom.**

(57) The amount of liquid products obtained by coking petroleum residua is increased by adding iron as such or in an iron-forming compound form thereof or as iron oxide to the residua before coking. The coke produced is highly active and can be readily converted to methane by contacting it with molecular hydrogen.

EP 0 031 697 A2

COMPLETE DOCUMENT

- 2 -

0031697

The present invention relates to an improvement in the known technique for coking various petroleum residua and further to a technique for forming methane from the coke so produced.

There are basically only two ways to upgrade petroleum residua. One is by catalytic cracking or hydrocracking. This technique suffers from at least two disadvantages, one of which is that comparative high pressures are required. The other disadvantage is that the pores of the catalyst rapidly become plugged both with coke and with metals present in the residua thus causing the catalyst to lose its activity rapidly.

The second method of upgrading petroleum residua is by coking. A disadvantage associated with this technique is that the yield of the more valuable liquid product (i.e. distillates) is more or less fixed. Furthermore, if the residua contains any significant amount of metals or sulphur, the coke product has value only as a fuel.

In our European Patent Application 80303445.3 filed 30th September 1980 a technique for increasing the amount of liquid product yields in a conventional coking operation is described. In this technique, the feed comprises a mixture of petroleum residua and shale oil or a shale oil fraction. In addition, a "hydrogen" catalyst, which may be a hydrogen transfer catalyst such as iron pyrites and alkaline iron oxide, are included in the reaction mixture. Although the yields of desired liquid products are increased, this procedure is limited to using a shale oil material as part of the feed and hydrogen transfer catalysts, or other "hydrogen" catalysts as therein defined. In addition, it is necessary in accordance with that process to feed hydrogen to the reaction system.

Accordingly, it is an object of the present invention to provide a simple and straight-forward technique for coking various petroleum residua which is capable of providing greater than expected yields of desired liquid product compared with conventional coking operations.

We have found that this object and others may be achieved by including in the petroleum residuum subjected to coking iron in the form of iron metal, and iron compound decomposable to form iron or iron oxide. In accordance with the present invention, it has been found that the presence of the iron causes a significant increase in the amount of liquid product produced during coking together with a concomitant decrease in the amount of coke produced. Moreover, it has been further found that the coke obtained in this manner is a highly reactive material and readily converts to valuable methane when contacted with hydrogen.

Thus the present invention provides a process for producing coke wherein a petroleum residuum is heated to elevated temperature in a non-oxidizing atmosphere to produce coke and a liquid byproduct, characterised in that the petroleum residuum contains an iron component which is iron metal, a compound decomposable to yield elemental iron at the coking temperature and/or an alkali metal free iron oxide.

Petroleum Residuum

As the petroleum residuum useful in accordance with the present invention, any petroleum derived refinery stream capable of undergoing any significant coking can be used. Such materials can be described as petroleum-derived refinery streams in which a minimum of 80% by weight boils above 600°F (315°C). Preferred petroleum residua are those in which a minimum of 80% boils above 700°F (371°C), most preferably 1,000°F (537°C).

For example, a preferred petroleum residuum is the distillation residue recovered as the bottom stream from a conventional vacuum distillation column. This material, commonly known as vacuum tower bottoms, has a boiling range such that a minimum of 80% boils above 1,000°F (537°C).

Another petroleum refinery stream that can be used as the petroleum residuum is the bottoms product of an atmospheric distillation column, which is normally characterised as having 80% boiling above 700°F (371°C). Still another petroleum refinery stream which is ideally suited as the petroleum residuum in accordance with the present invention is a decanted oil which is the bottoms product produced by distilling the effluent of a fluid catalytic cracker. As is well known, decanted oil is a highly aromatic material having a low API gravity and a boiling range such that 80% boils above 600°F (315°C), preferably 700°F (371°C) and is ideally suited for use as a coker feed.

Moreover, in accordance with the present invention, the petroleum residuum may be composed at least in part of organic matter derived from non-petroleum sources. For example, the petroleum material may include some bitumen from tar sands and/or pulverised coal. Essentially any amounts of bitumen can be included in the petroleum residuum while the amounts of pulverised coal in the petroleum residuum should be no more than about 50% by weight of the coal and petroleum residuum.

Iron Component

In accordance with the invention, an unexpected increase in the amount of liquid products produced during coking of various petroleum residua is obtained when iron is included in the petroleum residuum. The form of the iron to be used as the iron component can be either iron metal, a compound which thermally decomposes to yield elemental iron at coking temperatures or iron oxides. If the iron component is metallic iron, the metallic iron can be present in any form, although it is preferable that it be as finely divided as possible. Steel wool is a very effective form of metallic iron for this purpose, but other form of metallic iron such as steel shot, iron powder and so forth can also be used. Examples of compounds which decompose to yield elemental iron at coking temperatures are iron acetate, iron nitrate iron carbonate, iron thiosulphate and organometallic compounds such as iron naphthenate, iron oxalate and iron tartrate. Iron oxides which can be used as the iron component are any iron oxide, e.g. FeO, $Fe_2O_3$, and $Fe_3O_4$. It is preferable that any iron oxide used be free or essentially free of alkali metal ions so as to distinguish over the hydrogen transfer catalysts employed in our European Patent Application 80303445.3. In other words, in the invention of that application the iron-containing compounds used as catalysts are hydrogen transfer agents. While iron-containing compounds that are hydrogen transfer agents can be used in the present invention, iron-containing compounds which are not hydrogen transfer agents such as metallic iron and non-alkali metal-containing iron oxides can also be used.

Amount of Iron Catalyst

The amount of catalyst employed in the inventive process is not critical and can vary between wide limits. From an economic feasibility standpoint, the amount of catalyst should be no more than about 10 weight percent based on the weight of coker feed, and consequently the amount of catalyst in the feed material will normally be between greater than 0 and 10 percent by weight. The preferred amount of catalyst is about 1 to 7 percent with 3 to 4 percent catalyst being more preferred.

Coking Procedure

In accordance with the present invention, the coking operation is accomplished in the same way under the same conditions as conventional prior art coking procedures. Thus, in commercial operation the process is normally conducted in a semi-batch mode with the feed stream being continuously fed to the coker and liquid products continuously withdrawn from the coker. Coking of the feed continuously occurs in the coker until the coker is substantially full of coke, at which time the operation is terminated and coke is then removed from the coker.

The operating conditions for the coking operation, as indicated above, are conventional. For example, coking can be conducted at any conventional temperature such as from 600°F (315°C) to 1,000°F (537°C) preferably about 875°F (468°C). Also, conventional pressures, e.g. atmospheric pressure, can be used. Moreover, the feed stream can be heated while in the coker, although it is preferable to supply all the heat necessary for coking by preheating the feed stream in a furnace or other suitable device prior to entry of the feed stream into the coker.

In one embodiment of the invention, the coking operation is accomplished at a slightly elevated pressure, e.g. 1.1 to 5, preferably 2 to 4 atmospheres, in an atmosphere of carbon monoxide. It has been found that higher yields of the desired liquid products can be obtained when a carbon monoxide atmosphere is employed. The coking operation can, however, be carried out at elevated or atmospheric pressure in the presence of other non-oxidising atmospheres such as for example, carbon dioxide, nitrogen, etc. Hydrogen, however, is not fed to the coker during the coking operation of the invention.

Mixing

It is preferred that the coking operation be carried out so that the catalyst is at least partially mixed with the feed material undergoing coking. In this regard, it has been noticed in using a laboratory scale batch coker that the catalysts will normally settle to the bottom of the coker if the liquid therein is quiescent. Thus, if coking is accomplished in a strictly batch operation, it is preferable to mix the liquid in the coker during the coking operation so that the catalysts will be distributed throughout the mass of the liquid undergoing coking. Mixing can be accomplised by any conventional means such as using a mechanical mixer or passing an inert gas through the liquid. Preferably, carbon monoxide or analog can be employed for the purpose of mixing since this will automatically yield the advantage of improved liquid yields as described above.

Commercially, coking is usually accomplished in a semi-batch operation wherein liquid feed is continuously fed to a "delayed coker" and liquid products continuously removed from the coker. The liquid fed in the coker during the coking operation continues to be converted to coke and liquid product until the coker substantially fills with solid coke at which time the coking operation is terminated. In such an operation, feeding the liquid feed to the coker inherently causes enough mixing to provide resonable distribution of the catalyst in the liquid feed being coked.

Methanation

A further aspect of the invention is that the coke produced by the above procedure is highly reactive to form methane when contacted with hydrogen. In addition to molecular hydrogen, gas mixtures containing molecular hydrogen (e.g. synthesis gas) can be used as can compounds which decompose at methanation temperatures to produce molecular hydrogen such as, for example, iron carbonyl. The temperature for methanation can vary widely and is normally between about 800°F (426°C) and 1,500°F (815°C), preferably about 1,200°F (649°C). There is essentially no criticality in the methanation temperature, except that at temperatures significantly below 800°F (426°C) the reaction proceeds at such a low speed as to be economically unfeasible while at temperatures above 1,500°F (815°C) are not only unnecessary, but unduly deleterious to the coking vessel. The hydrogen flow rate can also vary widely and depends primarily on ease of operation and economic considerations. Generally, flow rates on the order of 0.005 to 0.1000 SCFM per pound of coke, preferably 0.002 to 0.050 SCFM per pound of coke, (0.0031 to 0.0062 standard cubic metres per minute per kilogram of coke, preferably .000124 to 0.0031 standard cubic metres per minute per kilogram) are employed. While methanation can be accomplished anywhere, it is most convenient to carry out methanation in the coker by simply feeding hydrogen thereto when appropriate. Any unreacted hydrogen can be recovered and recycled.

The following examples illustrate the invention:-

In these examples, coking was accomplished in a batch operation using a laboratory scale coker (mini-coker) composed of a carbon steel reaction vessel defining a cylindrical reaction compartment having a diameter of 4 inches (10 cms) and a height of 21 inches (53 cms). A feed line was connected to the bottom of the mini-coker for the introduction of feed material and an exit line was attached to the top of the mini-coker for withdrawl of gaseous and liquid products during the coking operation. In addition, another feed line was attached to the bottom of the mini-coker for the introduction of carrier gases for the purpose of mixing the contents of the mini-coker . In order to prevent condensation of the liquid product in the outlet line, the outlet line was heated to a temperature of 650°F (343°C) during the coking operation. In each example, 2,000 gms. of feed was charged into the mini-coker and the pressure in the mini-coker maintained at 25 psig and (1.75 kg/cm$^2$ gauge) the mini-coker heated using the thermal cycle shown in the following Table 1. Changes in temperature were made as rapidly as possible. In a typical coking cycle, the total elapsed time ranged from 400 to 450 minutes.

TABLE 1

Time at Temperature in the Mini-Coker

| Temp., °F | °C | Time, Minutes |
|---|---|---|
| 600 | 315 | 45 |
| 800 | 426 | 45 |
| 900 | 482 | 30 |
| 1,000 | 537 | 30 |
| 1,100 | 593 | 30 |
| 1,200 | 648 | 90 |

In each example, the feed was composed of vacuum tower bottoms having an API of 11.90 and containing 1.25% sulphur and 0.36% nitrogen, and the liquid products obtained were composed predominantly of kerosene, naphtha and gas oil. In those tests that were continued through a methanation cycle, the coke drum temperature was maintained at 1,200°F (648°C) and the overhead line was maintained at 650°F (343°C). The volume of off gas was measured and samples were taken at regular intervals for analysis. The carrier gas was deleted from the gas analysis for material balance purposes. In those tests in which steel wool was used as the catalyst, its weight was not included in the material balance calculations. Also, during coking a carrier gas was fed to the coker at 0.036 SCFM, (.00223 standard cubic metres per minute) and during methanation hydrogen gas was supplied to the mini-coker at 0.36 SCFM (.00223 standard cubic metres per minute).

Unless otherwise indicated, all results are given in weight percent based on the weight of the vacuum tower bottoms feed.

Comparative Example A and Example 1

In comparative Example A, coking of the vacuum tower bottoms without the presence of a catalyst was accomplished in the manner described above. In Example 1, iron naphthenate was used as the coking catalyst, the iron naphthenate being supplied in the form of an iron naphthenate solution in kerosene containing 6 weight percent iron. The conditions for coking and the results obtained are set forth in the following Table II.

0031697

## TABLE II

| | Conditions | | | Products | |
|---|---|---|---|---|---|
| Example | % Cat | Run Time (Hours) | Carrier Gas | % Coke | % Liquid |
| Comp A | 0.00 | 7.20 | $N_2$ | 36.68 | 49.61 |
| 1 | 2.43 | 7.45 | CO | 31.31 | 56.56 |

| | Products | | | | | | | | | |
|---|---|---|---|---|---|---|---|---|---|---|
| | % Gas | | | | | | | | | |
| Example | $H_2$ | $CO_x$ | $H_2S$ | $C_1$ | $C_2$ | $C_3$ | $C_4$ | $C_5$ | $C_6+$ | Total |
| Comp A | 0.08 | 0.18 | 0.20 | 3.32 | 2.59 | 1.94 | 0.75 | 0.12 | 0 | 9.18 |
| 1 | 0.14 | 0.11 | 0.02 | 2.25 | 2.55 | 1.57 | 0.91 | 0.39 | 0 | 7.94 |

Example 2

Example 1 was repeated except that the iron naphthenate content was 2.50%, and the total run time was 8.67 hours. At the 7.67 hour mark, methanation was begun by changing the gas feed to the mini-coker to $H_2$. Methanation was continued for 1 hour. At the end of methanation, it was found that the coke yield was 30.71%, the liquid yield was 57.26% and the total gas yield was 8.10 weight percent. Of this gas yield, 6.87% has been produced during coking and was composed of 0.08% $H_2$, 0.08% $CO_2$, 0.02% $H_2S$, 2.01% $C_1$, 2.38% $C_2$, 1.66% $C_3$, 0.47% $C_4$, 0.17% $C_5$, and 0% $C_6+$, while 1.23% was produced during methanation and contained 0.01% $CO_x$, 0.00% $H_2S$, 0.62% $C_1$, 0.49% $C_2$, 0.05% $C_3$, 0.06% $C_4$, 0.00% $C_5$ and 0% $C_6+$.

Example 3

Example 1 was repeated except the catalyst was composed of 4.13% unshredded steel wool and the total run time was 7.17 hours. It was found that the coke yield was 34.26%, the liquid yield was 52.39% and the gas yield was 8.81% and composed of 0.10% $H_2$, 0.14% $CO_x$, 0.02% $H_2S$, 2.50% $C_1$, 2.71% $C_2$, 1.77% $C_3$, 0.99% $C_4$, 0.58% $C_5$, and 0% $C_6+$.

Example 4

Example 2 was repeated except that 3.90% unshredded steel wool was used as the catalyst, the total run time was 14.40 hours and methanation was begun at the 7.25 hour mark. It was found that the coke yield was 27.46%, the liquid yield was 54.49% and the total gas yield was 14.01%. 10.99 of this total gas yield was produced during coking and was composed of 0.13% $H_3$, 0.32% $CO_x$, 0.04% $H_2S$, 3.69% $C_1$, 3.10% $C_2$, 2.41% $C_3$, 0.54% $C_4$, 0.76% $C_5$, and 0% $C_6+$. 3.02% of this total gas yield was produced during methanation and was composed of 0.06% $CO_x$, 0%, $H_2S$, 2.01% $C_1$, 0.66% $C_2$, 0% $C_3$, 0.11% $C_4$, 0.18% $C_5$, and 0% $C_6+$.

From the foregoing experiments, it can be seen that the amount of liquid produced during coking when iron

0031697

is included in the coking feed is significantly greater than when no iron is included. In addition, it will be noticed that this advantageous result is accomplished without supplying hydrogen to the coking drum during the coking operation. Also, it will be further appreciated that an off gas containing a high concentration of methane is produced by the methanation procedure.

- 13 -

0031697

CLAIMS:-

1. A process for producing coke wherein a petroleum residuum is heated to elevated temperature in a non-oxidising atmosphere to produce coke and a liquid byproduct, characterised in that the petroleum residuum contains an iron component which is iron metal, a compound decomposable to yield elemental iron at the coking temperature and/or alkali metal free iron oxide.

2. A process as claimed in claim 1 characterised in that the component is metallic iron.

3. A process as claimed in claim 1 characterised in that the component is a compound decomposable to yield elemental iron at the coking temperature.

4. A process as claimed in claim 3 characterised in that the compound is iron naphthenate.

5. A process as claimed in claim 1 characterised in that the iron component is an iron oxide.

6. A process as claimed in any of claims 1 to 5 which further includes contacting said coke with hydrogen to produce methane.

7. A process as claimed in claim 7 characterised in that the hydrogen is contacted with coke at a temperature of from 800°F (426°C) to 1,500°F (815°C).

8. A process for the production of methane by contacting coke with hydrogen characterised in that the coke has been prepared by a process a claimed in any of claims 1 to 5.